# EUROPEAN PATENT APPLICATION

(11) **EP 4 506 910 A1**
(43) Date of publication of application: **12.02.2025**
(21) Application number: 23191211.4
(22) Date of filing: 11.08.2023
(51) Int. Cl.: G06V 40/18, A61B 3/117, G06T 7/00

(54) **METHOD AND DEVICE FOR AUTOMATIC CLASSIFICATION OF CATARACTS**

(71) Applicant: TeleMedC GmbH, 20095 Hamburg (DE); Universitätsklinikum Hamburg-Eppendorf, 20246 Hamburg (DE)
(72) Inventor: Pérez de Olaguer, Nicolás, 20095 Hamburg (DE); Lleó, Albert, 20095 Hamburg (DE); Xiao, Di, 20095 Hamburg (DE); Skevas, Christos, 20095 Hamburg (DE)
(74) Representative: RGTH

(57) **Abstract**

A computer-implemented method (100) for automatic classification of cataracts is suggested which includes a receipt (101) of multiple cross-section images (50) of an anterior eye segment including the intraocular lens, a segmentation (102) of a lens region from each image (50) to obtain respective lens objects (51), a recognition (107) of cataract patterns in the lens objects (51) and a classification (108) of a cataract based on the recognized cataract patterns.

## Description

The present invention relates to a method for automatic classification of cataracts as well as to a respective device according to the independent claims.

### Prior art

Cataract is the leading course of blindness and accounts for around 33 % of all occurrences of visual impairment. Thus, the cataract cure ratio can be seen as a significant indicator of a country's development stage. The current diagnosis of cataracts relies on subjective visual inspections by ophthalmologists who observe the anatomical changes occurring in the eye's lens based on ophthalmic images. However, this manual diagnosis approach is susceptible to mistakes, time consuming, subjective and expensive which makes it especially difficult to be used in developing countries or rural communities where qualified clinicians are view.

### Objective, solution, advantages

The objective of the present invention is to improve the accuracy and consistency of cataract diagnosis as well as reducing the time and effort required.

The present objective is solved by a computer-implemented method for automatic classification of cataracts which includes receiving multiple, preferably two-dimensional gray-scale, cross-section images of an anterior eye segment including the intraocular lens. The method further includes segmentation of a lens region from each image to obtain respective lens objects, recognition of cataract patterns in the lens objects and classification of a cataract based on the recognized cataract patterns.

Cataracts are caused by the loss of crystalline lens transparency commonly referred to as the clouding of the eye lens. The clouding appears when the protein within the lens clumps together, often leading to vision loss and other potential complications. Various factors can trigger this condition, such as developmental issues, physical drama, metabolic disorders, alterations induced by certain medications as well as the natural aging process.

In particular, multiple cross-section images of the same eye are received. Each of them includes the intraocular lens. The images which are received are preferably Scheimpflug images and portrait the anterior eye segment from different angles. Scheimpflug images provide a section of the lens, evenly focused from the anterior to posterior capsule.

Scheimpflug images are taken by following the Scheimpflug principle, first introduced by Theodor Scheimpflug. It describes an optical imaging condition, which allows documentation of an obliquely tilted object with the maximally possible depth of focus and minimal image distortion. The increased depth of field or plane of sharp focus in Scheimpflug imaging is achieved by changing the relative plane of film to the camera lens. In a regular camera, the film plane and the lens plane are parallel to each other, resulting in a plane of focus which runs parallel to these. Slit-lamp photographs, on the other hand, provide an image with the best focus corresponding to only a part of the lens either the anterior or posterior capsule, while other parts of the image are not focused. Out of focus images cannot be accurately analyzed for lens density.

Especially, the Scheimpflug images have been retrieved by a Pentacam Scheimpflug camera (preferably by Oculus, Wetzlar, Germany). These are taken from various angles around the eye. The camera capturing the images, especially the Pentcam, circumnavigates the anterior eye segment capturing images, preferably an image every 14.4°, thereby providing a full circle view around the eye. The output of the camera which is the input for the present method is a series of images. These images can have a dimension of 1.200 x 620 pixels in gray scale values, signifying a single channel with integer values ranging from 0 to 255. Provided by the Pentacam, the images can be identified by a specific naming system incorporating patient's identification, the eye under examination, the date and the image frame.

After receipt of the multiple cross-section images the images can be renamed. Further, it can be checked whether the data set is complete. Using Scheimpflug images, the images for each eye could be counted. It could be checked whether a predefined minimum number is present, before further steps of the method are completed. Further, if more than the predefined number is present, a subset could be chosen, e.g., based on the quality of the images. If less images are provided, images could be generated or duplicated to reach the pre-defined number.

The present invention comprises segmenting a lens region from each image. This means that the region containing the lens is identified and respectively segmented. In other words, the region containing the lens is isolated from the rest of the received images. The resulting images which are focused on the lens are referred to as lens objects in this application.

As a next step, the method comprises recognizing cataract patterns in the lens objects. The cataract patterns in the lens objects are recognized based on the change in density as an effect of the loss of crystalline lens transparency. In other words, the partial clouding of the eye lens as local opacities is recognized as cataract patterns.

Cataracts can generally by classified into three types based on the locations of the cataract patterns: Nuclear sclerotic, cortical and posterior subcapsular cataract.

Nuclear sclerotic cataract refers to the gradual clouding of the central part of the lens and can further relate to a progressive hardening of the lens nucleus. Cortical cataract can appear in a spoke-like pattern from the outside border of a lens towards a center. It is a radially oriented opacity. Posterior subcapsular cataracts are granular opacities which appear like little breadcrumbs or sand particles strewn beneath the lens.

The size, the position as well as the number of cataract patterns in the lens objects can be identified. Especially, the method maps the cataract patterns of the lens.

Based on this, a cataract can be classified. Especially, the method is configured to identify which type of cataract, namely nuclear, cortical and posterior sub capsular cataract is present. For example, the output of the method could be a probability for each type of cataract. The probability indicates with which certainty this type of cataract is present. Thus, the output can consist of three probability values, which can be between 0 (0 percent; probability as low as possible) and 1 (100 percent; probability as high as possible). The method can provide the output as an array of the probability values, wherein the probability value for each type of cataract is independently derived. The array maps each probability uniquely to one of the predefined cataract categories.

As a further step, the method can comprise grading the cataract based of the recognized patterns. The grading can be based on the severity of the cataract patterns, especially their number, their sizes, and the respective opacity. Relating to a predefined grading system, the method can output a set of probabilities for each grade wherein again the values add up to 1 (or 100 %). Thus, the values indicate the estimated likelihood that a respective grade is present in the respective received images.

The output is compliant with the LOCS III grading system or the WHO grading system which both provide a standard and objective way to grade the severity of cataracts. For example, there could be predefined grades 0 to 3, where 0 means non-referable and 1 to 3 relate to the severity (1 being the least severe and 3 the most severe). An output could be [0.1, 0.8, 0.055, 0.045]. The numbers are allocated to the grades. This output would thus indicate that most likely there is a cataract present with grade 1.

The output can be showcased via a user interface or exported in a structured format such as for example see CSV or JSON-file for seamless integration with electronic health record systems.

The output empowers ophthalmologists to make more informed decisions regarding the optimal treatment path for the patient. Based on the classification and especially on the grading the method can output a recommendation whether the images are to be reviewed by an eye specialist or whether this is not necessary. In the latter case, a detailed report can be output to the eye specialist including the cataract specification in grading results. The provision of the recommendation whether the reviews necessary can be dependent on the likelihoods regarding the different grades. A respective threshold can be predefined. If - for example - a likelihood regarding a grade is larger than 0.8, thus 80 %, it can be decided that the determination of the grade is sufficiently precise to recommend that a further review is not necessary. If, however, each likelihood is lower than 80 % it can be suggested at a review is necessary.

Especially, the segmentation is achieved by applying a bounding box technique. A bounding box position can be predicted. Especially, before predicting the bounding box position, image features from the lens objects can be extracted. The lens region can be encapsulated with the bounding box and the pixel coordinates of the bounding box can be regressed. The coordinates of the bounding box can be extracted. Based on these coordinates, the lens region can be cropped, in other words the lens region based on the extracted coordinates of the bounding box is cut out. The cut-out image is the lens object and can be resized for the following steps of the method. For example, the lens objects can have dimensions of 300 x 300 pixels after resizing. Further, if the lens object contains some residual part of the iris, these can be removed. This is achieved by a recognition of these parts based on a differentiation in gray -scales of the residual iris parts and the lens.

Before the recognition of cataract patterns, multiple images are consolidated into a single image comprising multiple channels. Thus, regarding Scheimpflug images, 25 images can be stacked as a single image with 25 channels. A consolidated image can comprise 25 distinct channels each corresponding to one of the 25 patient-specific images and have a dimension of 300x300 pixels with a gray-value range of 0 to 255.

Especially, the segmentation can be conducted by using a neural network, especially a convolutional neural network (CNN) can be used.

Especially, the CNN has been trained on manually labeled cross-section images. The core of the CNN can be based on the VGG16 model, which is recognized for a simple architecture and efficiency in image analysis.

The neural network can comprise 3x3 convolutional layers stacked on top of each other and can be deep with 16 layers that include convolutional and fully connected layers. This deep structure aids in learning more complex image features and is especially suitable for detecting the lens in the images since hierarchical features of the image are extracting by the stacking of the convolutional layers. The features relate to low level features such as for example straight lines, edges etc. in the first layers to complex features towards the deeper layers. At the end of the CNN, a fully connected layer can be placed for outputting the coordinates of the bounding box. The used CNN can consist of four neurons each one corresponding to a target value of the bounding box. Either the coordinates itself are extracted or the x and y coordinate of one point of the bounding box as well as the width and the height of the bounding box can be extracted. The CNN has been trained on manually labeled images in which the lens within the cross-section images of the anterior eyes is labeled. Thus, by the training process with training data the convolutional filters and weights have been modified to operate on unseen data and the network learned the context of the image in the specific region where the lens is located.

In detail, the network can have the following last layers: A dense layer with 128 units and the activation function Rectified Linear Unit (ReLU). The output from the previous layer, which is a flattening layer, is fed into this layer. The output of the dense layer is then passed to another dense layer, this time with 64 units, also using the ReLU activation function. The process is repeated with a subsequent dense layer of 32 units, again with ReLU activation function, and to another dense layer consisting of the above mentioned 4 units or neurons, but this time the activation function used is Sigmoid. In other words, this block of code represents a chain of fully connected layers, decreasing in size from 128 to 64, then 32 and finally 4 units with the final layer outputting the predicted bounding box coordinates.

Before the recognition of the cataract patterns and the respective classification and possible grading, the histogram of each lens object can be equalized to increase their contrast. Especially, an image processing algorithm can be applied.

In particular, an adaptive histogram equalization technique can be applied, most preferred a contrast limited adaptive histogram equalization technique. Unlike traditional histogram equalization, a contrast limited equalization histogram equalization technique operates on small, localized region of the image, in this case the lens object. These localized regions can be referred to tiles. This helps preserving local contrast and reduce noise amplification. Especially, a clip limit parameter is set to three at most and controls the contrast amplification, preventing it from exceeding a specified limit to reduce noise. In other words, the contrast limited adaptive histogram equalization takes care of the over-amplification of the contrast as compared to adaptive histogram equalization, since it works on smaller regions of the image - the tiles - as explained above, rather than the entire image. The basic idea is to limit the contrast, especially in homogeneous areas, to avoid amplifying noise which might be present in the image. In other words, the technique improves the local contrast and enhances the definition of edges.

As a first step, the input lens objects can be divided into small blocks - the so-called tiles. For example, the image can be divided into blocks or tiles of 8 pixels. The image can be padded, if the image dimensions are not an exact multiple of the tile size. These tiles are then treated as small individual images. Histogram equalization is applied to each such tile independently. The equalization is done by distributing the amounts of brightness levels (in other words the number of bins regarding all brightness levels) equally. In doing so, the contrast can be limited. If any histogram bin is above a specified contrast limit, those extra pixels are clipped off and distributed uniformly among all other bins before applying the histogram equalization. The contrast, especially in homogenous areas, is thus limited to avoid amplifying any noise that might be present in the image. Finally, the histogram equalization is applied on each block, taking into account the contrast limit. As a result, the histogram of each of these blocks is almost flat, meaning that the lens object as the sum of the tiles has equal amounts of all brightness levels. As an effect, each tile's contrast is enhanced. However, this can result in borders between tiles becoming visible. A bilinear interpolation can be applied between the tile corners to remove these artifacts.

As a last step the sub-image, thus the tiles are then combined back into a final image. The result is an enhanced contrast and better visibility of details, particularly in areas previously too dark or too light, which helps in recognizing the cataract patterns. It further prevents noise from the amplified and relatively homogeneous regions of the image.

The classification, preferably including the recognition of cataracts patterns, and/or the grading can be conducted by using a neural network, especially a CNN, which can be previously trained on manually labeled cross-section images. The CNN can be trained to identify, categorize and grade the cataract patterns evident in the lens objects.

Especially, the neural network uses a compound scaling method scaling the depth, in other words the network layers, the width, in other words, the number of channels, and the resolution of the network jointly in a balanced way which leads to better performance. Thus, a compound scaling factor is computed used to scale all three dimensions together which ensures a higher accuracy in the classification task with lower computational costs compared to other networks.

The core of the neural network can be based on the EfficientNet neural network which optimizes model scaling. All batch normalization layers (which help to standardize the inputs to each layer of the network) can be replaced with group normalization layers. These group normalization layers are a variation of batch normalization that divides channels into smaller groups and normalizes the features within each group. Then, a global average pooling operation is performed by having a respective pooling layer. This operation calculates the average output of each feature map in the previous layer which significantly reduces the model's size and helps to prevent overfitting. A group normalization layer is added to normalize these pooled features, and a dropout layer follows to randomly ignore a certain fraction of neurons during training, further reducing overfitting. A dense layer with an 'elu' activation function comes next. The size of this layer (the number of neurons) is equal to the target size which should match the number of classes in a classification problem. Finally, if the model is set to perform regression, a single-neuron dense layer with a 'linear' activation function is added. This layer will predict a single continuous output.

The present method provides an automated computer-implemented way to classify and grade cataract without the need for human intervention. A reduction in both inter- and intra-observer variability as well as a faster and more efficient assessment process is achieved. Overall, the standard of care provided to patients is elevated. The technology has the potential to significantly improve the standard of care by providing a more objective and efficient method for diagnosing and grading this very common eye condition. The method can help in increasing the rate of diagnosis in developing countries in which the rate of untreated visual impairment is much higher than in developed countries. Overall, the standard of eye care for patients with cataracts is improved by providing a more objective and efficient method which is universally accessible to all healthcare providers regardless of the expertise levels.

In detail, the advantages are the following:
Increased objectivity and consistency: Unlike traditional manual diagnosis which is tending to observer variability and errors, the invention ensures objective and consistent evaluation of cataracts. The complex patterns of the cataract are interpreted by the computer-implemented method, preferably a CNN, providing the necessary insights and reference points. The simplified image interpretation is sufficient for the system as the primary aim is to provide a consistent and comprehensive assessment of the cataract's severity.

Enhanced architecture: The architecture of the software-based system is designed to facilitate the processing of the data. The system accepts standard Scheimpflug images as input, pre-processes the images to ensure data quality and consistency, and outputs a classification and grade for the cataracts present in the image. This process removes the need for a manual evaluation by a clinician, and enables faster and more efficient detection and grading of cataracts, thereby allowing for more timely and appropriate treatment for patients.

Improved user interface and output integration: The output can be displayed in an easily digestible format regarding the classification and the grade for cataracts present in the image. This allows ophthalmologists to make more informed decisions about the appropriate treatment for the patient. The output can be presented on a user interface or exported in a structured format as a report. This not only improves the decision-making process but also enhances the integration of the system with existing healthcare infrastructures.

Overall, the invention significantly enhances the standard of care for patients with cataracts by providing a more objective, reliable, and efficient method for diagnosing and grading cataracts. This technology has the potential to revolutionize eye care, particularly in developing countries or rural communities where access to qualified clinicians might be limited.

In another aspect of the invention, it relates to a device for automatic classification of cataracts, wherein the device includes a segmentation submodule for - after receiving multiple cross-section images of an anterior eye segment including the intraocular lens - segmenting a lens region from each received each image to obtain a respective lens object. The device further includes a cataract classification submodule for recognizing cataract patterns in the lens objects and for classifying a cataract based on the recognized cataract patterns. The device is preferably configured to conduct a method described above.

Furthermore, the device can include a data preprocessing submodule for renaming received images and/or checking the completion of the received images. The segmentation submodule can apply a bounding box technique as explained above.

In addition, the device can have an image processing submodule for equalizing the histograms of the lens objects. The cataract classification submodule can also be configured to grade the cataract. Additionally, the device can comprise an output submodule for providing an output relating to predefined cataract types and preferably predefined cataract grades.

Overall, the device can consist of two modules, a first module comprising the segmentation submodule and further preferred the data preprocessing submodule and a second module which can comprise the cataract classification submodule and further preferred the image processing submodule and the output submodule.

The network regarding the segmentation can be trained as follows:
The CNN for the segmentation is especially trained on a data set of bi-dimensional single-channel images, in other words gray-scale images. This means that the depth of the images is 1, but the gray-scale is composed of 2 dimensions, x and y. The images are manually labeled by marking coordinates of four pixels which define the lens region and thus the bounding box. For training, the respective image as well as the corresponding coordinates which have been hand labeled are employed.

The training process spans approximately 20 epochs. It is a supervised learning algorithm since the data is manually labeled.

As a model, the VGG16 has been used and pretrained using the Imagenet database. It is useful to have the weights set to a specific starting point where the model training can converge easily for this specific task. The model was modified in the head of the network i.e., last layers, to the following:

First, a dense layer with 128 units is created and the activation function used is ReLU. The output from the previous layer, which is a flattening layer, is fed into this layer. The output of the dense layer is then passed to another dense layer, this time with 64 units, also using the ReLU activation function. The process is repeated with a subsequent dense layer of 32 units, again with ReLU activation function, and to another dense layer consisting of 4 units or neurons, but this time the activation function used is Sigmoid. In other words, this block of code represents a chain of fully connected layers, decreasing in size from 128 to 64, then 32 and finally 4 units with the final layer outputting the predicted bounding box coordinates.

A stochastic gradient descent and back progression is used to train the CNN aiming to minimize a predefined error function. In this case, the error function is the mean squared error (MSE), thus the difference between the coordinates predicted by the network and the actual coordinates which have been marked. Essentially, the error function serves as a distance measure for each pair of coordinates. Once trained, the CNN is used in inference mode, which means that it is not learning but is used for predicting the respective coordinates of the bounding box.

The network regarding the classification which preferably included the recognition of cataract patterns can be trained as follows:
The training can be conducted on more than 15.000 bi-dimensional single channel images. Before the training class weights are computed for each unique defined class in the training data set. The purpose of these class weights is to handle any imbalance in the training data set helping the model to pay more attention to underrepresented classes. This is achieved by a process that computes the "balanced" class weights for each unique class. The class weights computed earlier are used during the training process to give more importance to underrepresented classes, thus helping to improve the model's performance even when there is a class imbalance in the training data.

The training process can span 20 epochs. The model can use an optimizer with a specific learning rate especially 0.0002 to adjust the internal parameters and minimizing the loss.

The training is monitored with a condition known as early stopping. This means that if the model's performance on a validation set does not improve for a certain set number of epochs, for example 15, the training will stop early. This helps to prevent overfitting where the model performs well on the training data but poorly on new, unseen data. The learning is supervised.

The model used is the EfficientNet. The target size is equal to a number of unique classes indicating the number of output nodes needed in the final layer of the model. The model is designed to classify images into multiple classes. The dropout rate during training is specified in the parameters, which helps to prevent overfitting by randomly ignoring some neurons during the training process. The model is set as trainable, meaning its parameters will be updated during the training process. It is also important to note that all batch normalization layers (which help to standardize the inputs to each layer of the network) within this base model are replaced with group normalization layers. These group normalization layers are a variation of batch normalization that divides channels into smaller groups and normalizes the features within each group.

Then, a global average pooling operation is performed. This operation calculates the average output of each feature map in the previous layer which significantly reduces the model's size and helps to prevent overfitting.

A group normalization layer is added to normalize these pooled features, and a dropout layer follows to randomly ignore a certain fraction of neurons during training, further reducing overfitting.

A dense layer with an 'elu' activation function comes next. The size of this layer (the number of neurons) is equal to the target size which should match the number of classes in a classification problem.

Finally, if the model is set to perform regression, a single-neuron dense layer with a 'linear' activation function is added. This layer will predict a single continuous output.

Since the model is set to perform a classification task, four neurons dense layer with a 'linear' activation function is added. This layer will predict a set of probabilities for every of the possible classes, i.e., cataract grades.

The loss function used in this training process is the 'categorical cross-entropy' loss. It quantifies the difference between the true labels and the predictions made by the model. The goal of training is to minimize this difference.

### Brief description of the figures

The figures show in only schematic representation:
- Figure 1:: a flow diagram of a method for automatic classification of cataract;
- Figure 2:: a flow diagram of some steps of the method according to figure 1;
- Figure 3:: a device for automatic classification of cataract;
- Figure 4:: the data preprocessing submodule of the device of figure 3;
- Figure 5:: the segmentation submodule of the device of figure 3;
- Figure 6:: the image processing submodule of the device of figure 3; and
- Figure 7:: a flow diagram of some steps of the method according to figure 1.

### Embodiments of the invention

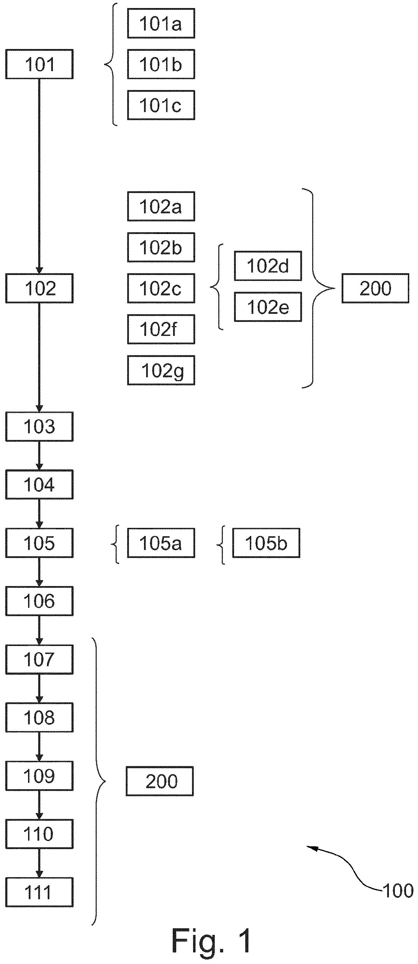

Figure 1 shows a method 100 for automatic classification of cataracts.

As a first step multiple cross-section images 50 of an anterior eye segment including the intraocular lens are received 101. The images can be renamed 101a, checked 101b for completion and images belonging to the same eye can be grouped 101c.

Further, a lens region from each image is segmented 102 to obtain respective lens objects 51. This step can be achieved by using 200 a neural network and can include applying 102a a bounding box technique. Image features can be extracted 102b and a bounding box position can be predicted 102c by encapsulating 102d the lens region with the bounding box and regressing 102e pixel coordinates of the bounding box. As a result, coordinates of the bounding box are extracted 102f and the lens region, in other words the lens objects, is cropped 102g.

Residual iris parts which can still be present in the lens objects 51 can be removed 103 and the lens objects 51 could be resized 104.

A respective histogram of the lens objects 51 can be equalized 105, especially by applying an image processing algorithm. Preferably, an adaptive histogram equalization technique is applied 105a. Most preferred, a contrast delimited adaptive histogram equalization technique is applied 105b. The contrast enhanced lens objects 51 can be consolidated 106 into a single image 52 with multiple channels.

Cataract patterns can be recognized 107 and a cataract can be classified 108 based on the recognized cataract patterns. Further, the cataract can be graded 109. The method 100 outputs 110 a classification and a grade for cataract present in the original images 50.

Based on the classification 108 and especially on the grading 109 the method 100 can output 111 a recommendation whether the images are to be reviewed by an eye specialist or whether this is not necessary. The provision of the recommendation whether the review is necessary can depend on the likelihoods assigned to the grades.

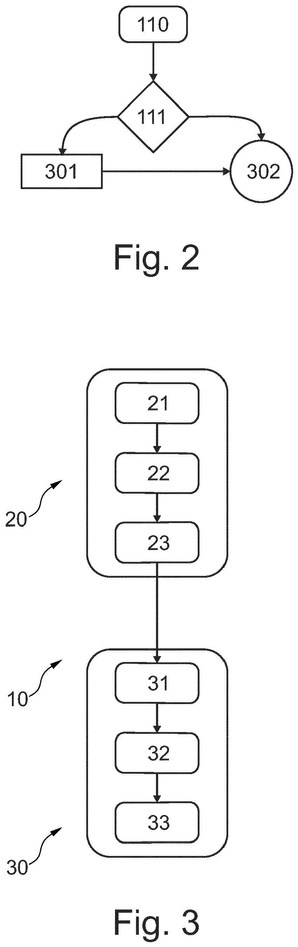

Figure 2 shows how after a recommendation is provided 111, a review 301 by an eye specialists can be conducted or not. In the latter case, a detailed report can be provided 302 directly to the eye specialist including the cataract specification and the grading results.

Figure 3 displays a device 10 for automatic classification of cataract. The device 10 consists of a first module 20 and a second module 30. The first module 20 comprises an input submodule 21 which receives two-dimensional gray-scale cross-section images of the anterior segment of the eye including the lens. Further, the first module 20 can have a data preprocessing submodule 22 as well as a segmentation submodule 23. The second module 30 has an image processing submodule 31, a cataract classification submodule 32 and an output submodule 33.

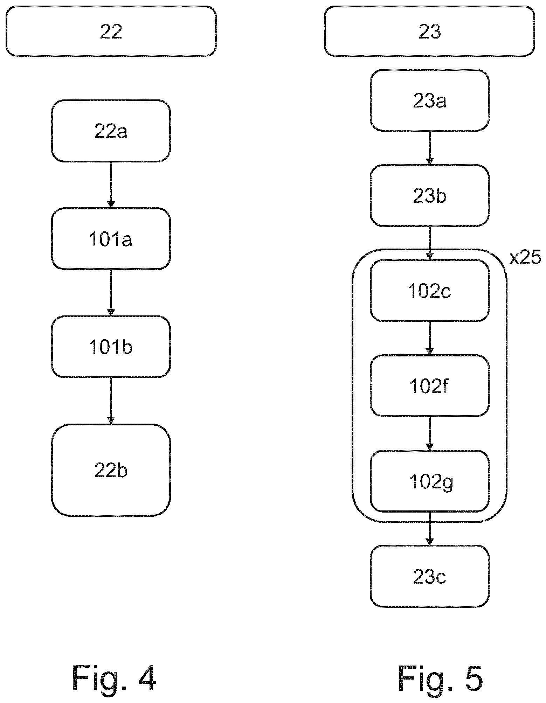

In figure 4 the data preprocessing submodule 22 of the device 10 of figure 3 is explained in detail. It has an input 22a in which it receives 101 the multiple images 51 of the anterior eye segment. These images 51 can be renamed 101a, checked 101b for completion and grouped 101c belonging to the same eye, before they are output 22b to the following submodule. If the image set is not complete, thus the number of the images per eye, do not reach a predefined threshold, the data preprocessing submodule 22 is configured to disregard the patient due to an incomplete data set and to notify the user respectively.

Figure 5 shows the segmentation submodule 23 of the device of figure 3 in detail. It has an input 23a as well as an output 23c. As inputs, the images from the data preprocessing submodule 22 are taken. If a neural network is used 200 for the steps of the segmentation it can be loaded 23b. Regarding each image of the eye, a bounding box position is predicted 102c and the coordinates of the bounding box are extracted 102f. The lens region defined by the bounding box is cropped 102g. As an output the lens objects 51 are fed to the second module 30.

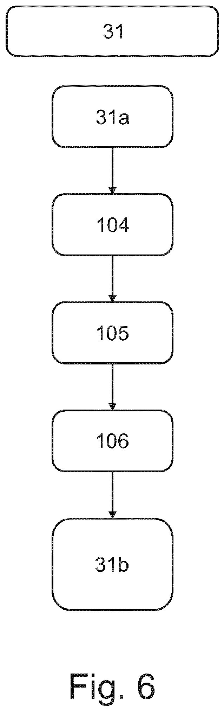

In figure 6 the image processing submodule 31 of the device of figure 1 is shown in detail. There is an input 31a as well as an output 31b. The lens objects can be resized 104 and their respective histograms can be equalized 105. Further, they can be consolidated 106 into a single image 52 with multiple channels. As an output, the single image 52 with multiple channels is fed to the cataract classification submodule 32.

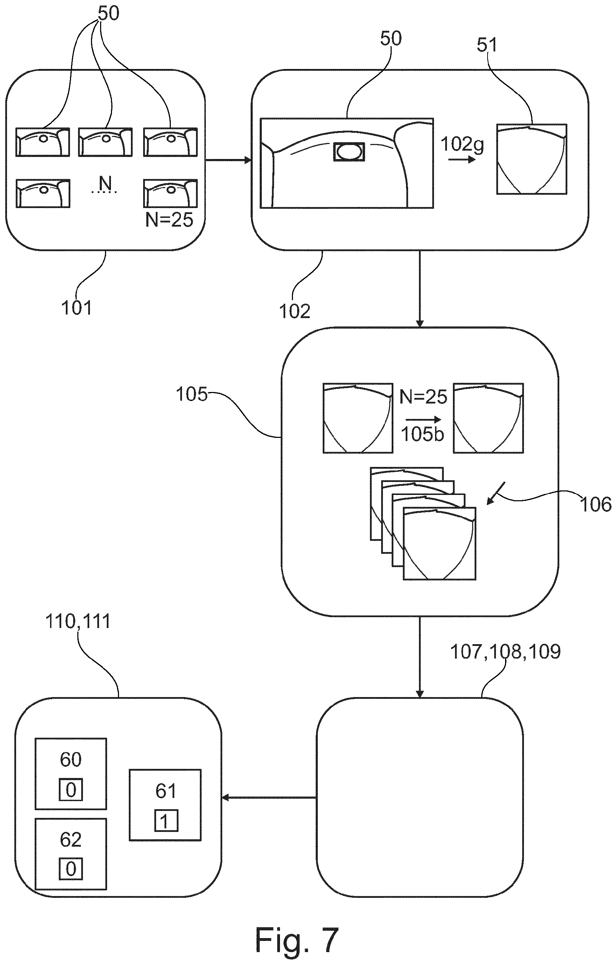

Figure 7 shows cross-section images 50 which are received 101. In this case, their number is 25, since they are Scheimpflug images. Figure 7 shows important steps of the method 100 for automatic classification of cataract 100 according to figure 1.

During the segmentation 102 an area including the lens is segmented and cropped 102g such that lens objects 51 are obtained. In the left part of the box relating to the segmentation step 102, namely in the image 50, a bounding box is pictured γ example.

Further, the histograms of the lens objects 51 can be equalized 105 by applying 105b a contrast limited adaptive histogram equalization technique. Cataract patterns can be recognized 107 and the cataract can be classified 108 and graded 109. As an output 110 a classification and a grade for the cataract present in the images can be provided. Further, a suggestion whether a review is necessary can be provided 111. In figure 7, three types of cataract, namely a first type 60, a second type 61 and a third type 62 are predefined, wherein the output is 0 for the first type 60, 1 for the second type 61 and 0 for the third type 62. Thus, the method in this example has evaluated that a cataract of the second type 61 is present.

### Reference signs

- 100: Method for automatic classification of cataract
- 101: Receipt of multiple cross-section images of an anterior eye segment including the intraocular lens
- 101a: renaming images
- 101b: checking completion of dataset
- 101c: grouping images of same eye
- 102: Segmentation of a lens region from each image to obtain respective lens objects
- 102a: applying a bounding box technique
- 102b: extraction image features
- 102c: predicting bounding box position
- 102d: encapsulating lens region with a bounding box
- 102e: regressing pixel coordinates of the bounding box
- 102f: extracting coordinates of bounding box
- 102g: cropping the lens region to obtain a lens object
- 103: Removal of residual iris part
- 104: Resizing the lens objects
- 105: Equalizing histograms of the lens objects
- 105a: Applying an adaptive histogram equalization technique
- 105b: Applying a contrast limited adaptive histogram equalization technique
- 106: Consolidation of the multiple lens objects to a single image with multiple channels
- 107: Recognition of cataract patterns
- 108: Classification of a cataract based on the recognized cataract patterns
- 109: Grading of a cataract based on the recognized cataract patterns
- 110: Output of a classification and a grade for a cataract present in the images
- 111: Providing a recommendation whether review is necessary
- 200: Using a neural network
- 301: Review by eye specialist
- 302: Providing a report on the results

- 10: Device for automatic classification of cataract
- 20: First module
- 21: Input submodule
- 22: Data preprocessing submodule
- 22a: Input
- 22b: Output
- 23: Segmentation submodule
- 23a: Input
- 23b: Loading neural network
- 23c: Output
- 30: Second module
- 31: Image processing submodule
- 31a: Input
- 31b: Output
- 32: Cataract classification submodule
- 33: Output submodule

- 50: Cross-section images
- 51: Lens object
- 52: Multi channel consolidated image

- 60: First Cataract Type
- 61: Second Cataract Type
- 62: Third Cataract Type

## Claims

1. A method (100) for automatic classification of cataracts,
**characterized in that** the method (100) is computer-implemented and includes:
- receipt (101) of multiple cross-section images (50) of an anterior eye segment including the intraocular lens,
- segmentation (102) of a lens region from each image (50) to obtain respective lens objects (51),
- recognition (107) of cataract patterns in the lens objects (51), and
- classification (108) of a cataract based on the recognized cataract patterns.

2. The method (100) according to claim 1,
wherein the images (50) are Scheimpflug images and portray the anterior eye segment from different angles.

3. The method (100) according to claim 1 or 2,
wherein the method (100) further comprises grading (109) the cataract based on the recognized patterns.

4. The method (100) according to any one of the preceding claims,
wherein before the recognition (107) of cataract patterns the multiple lens objects (51) are consolidated (106) into a single image (52) comprising multiple channels.

5. The method (100) according to any one of the preceding claims,
wherein the segmentation (102) is achieved by applying a bounding box technique (102a).

6. The method (100) according to any one of the preceding claims,
wherein histograms of the lens objects (51) are equalized (105) to increase their contrast.

7. The method (100) according to claim 6,
wherein an adaptive histogram equalization technique is applied (105a).

8. The method (100) according to claim 6 or 7,
wherein a contrast limited adaptive histogram equalization technique is applied (105b).

9. The method (100) according to any one of the preceding claims,
wherein the segmentation (102) is conducted by using (200) a neural network.

10. The method (100) according to any one of the preceding claims,
wherein the classification (108) and/or the grading (109) is conducted by using (200) a neural network.

11. The method (100) according to any claim 9 or 10,
wherein the neural network is a convolutional neural network (CNN).

12. The method (100) according to any one of claims 9 to 11,
wherein the neural network has been trained on manually labelled cross-section images.

13. A device (10) for automatic classification of cataracts,
**characterized in that**
the device (10) includes:
a segmentation submodule (23) for segmenting (102) a lens region from each image (50) to obtain a respective lens object (51) after receiving (101) multiple cross-section images (50) of an anterior eye segment including the intraocular lens, and
a cataract classification submodule (32) for recognizing (107) cataract patterns in the lens objects (51) and for classifying (108) a cataract based on the recognized cataract patterns.

14. The device (10) according to claim 13,
wherein the device (10) is configured to conduct a method (100) according to any one of claims 1 to 13.

## Amended claims

### Amended claims in accordance with Rule 137(2) EPC.

1. A method (100) for automatic classification of cataracts,
wherein the method (100) is computer-implemented and includes:
- receipt (101) of multiple cross-section images (50) of an anterior eye segment including the intraocular lens,
- segmentation (102) of a lens region from each image (50) to obtain respective lens objects (51),
- recognition (107) of cataract patterns in the lens objects (51), and
- classification (108) of a cataract based on the recognized cataract patterns,
**characterized in that**
the images (50) are Scheimpflug images and portray the anterior eye segment from different angles.

2. The method (100) according to claim 1,
wherein the method (100) further comprises grading (109) the cataract based on the recognized patterns.

3. The method (100) according to any one of the preceding claims,
wherein before the recognition (107) of cataract patterns the multiple lens objects (51) are consolidated (106) into a single image (52) comprising multiple channels.

4. The method (100) according to any one of the preceding claims,
wherein the segmentation (102) is achieved by applying a bounding box technique (102a).

5. The method (100) according to any one of the preceding claims,
wherein histograms of the lens objects (51) are equalized (105) to increase their contrast.

6. The method (100) according to claim 5,
wherein an adaptive histogram equalization technique is applied (105a).

7. The method (100) according to claim 5 or 6,
wherein a contrast limited adaptive histogram equalization technique is applied (105b).

8. The method (100) according to any one of the preceding claims,
wherein the segmentation (102) is conducted by using (200) a neural network.

9. The method (100) according to any one of the preceding claims,
wherein the classification (108) and/or the grading (109) is conducted by using (200) a neural network.

10. The method (100) according to any claim 8 or 9,
wherein the neural network is a convolutional neural network (CNN).

11. The method (100) according to any one of claims 8 to 10,
wherein the neural network has been trained on manually labelled cross-section images.

12. A device (10) for automatic classification of cataracts,
wherein
the device (10) includes:
a segmentation submodule (23) for segmenting (102) a lens region from each image (50) to obtain a respective lens object (51) after receiving (101) multiple cross-section images (50) of an anterior eye segment including the intraocular lens, and
a cataract classification submodule (32) for recognizing (107) cataract patterns in the lens objects (51) and for classifying (108) a cataract based on the recognized cataract patterns,
**characterized in that**
the images (50) are Scheimpflug images and portray the anterior eye segment from different angles.

13. The device (10) according to claim 12,
wherein the device (10) is configured to conduct a method (100) according to any one of claims 1 to 11.
